# EUROPEAN PATENT APPLICATION

(11) **EP 3 730 544 A1**
(43) Date of publication of application: **28.10.2020**
(21) Application number: 18855154.3
(22) Date of filing: 13.12.2018
(51) Int. Cl.: C08K 3/013, C08K 3/36, C08K 3/08, B29C 64/112, B33Y 10/00, B33Y 80/00, A44C 25/00, A01N 1/00

(54) **FREEZE-DRIED COMPOSITION**

(30) Priority: 19.12.2017 ES 201731549 U
(71) Applicant: Grid Interactive de Empresas, S.L., 28005 Madrid (ES)
(72) Inventor: NARBÓN PRIETO, Gabriel, 28005 Madrid (ES); SÁNCHEZ-GUITARD LÓPEZ-VALERA, Francisco Ramón, 28005 Madrid (ES); TERUEL SAIZ, Antonio, 28005 Madrid (ES)
(74) Representative: Pons
(86) International application number: PCT/ES2018/070804
(87) International publication number: WO 2019/122476

(57) **Abstract**

The present invention relates to a composition comprising resin, metal selected from among Au, Ag, Pt and Rh, graphene and a sample of biological material from a person, animal or plant, alive or dead. Furthermore, the invention relates to the use of said composition for the manufacturing of an emotional object, of keepsake or locket. The composition of the invention has the purpose of the creation of an emotional link between an object comprising said composition and the user who wants to acquire it, and where said composition has conductive properties for its digital use.

## Description

The present invention relates to a composition comprising resin, metal selected from among Au, Ag, Pt and Rh, graphene and a sample of biological material from a person, animal or plant, alive or dead. Furthermore, the invention relates to the use of said composition for the manufacturing of an emotional object, of keepsake or locket.

The composition of the invention has the purpose of the creation of an emotional link between an object comprising said composition and the user who wants to acquire it, and where said composition has conductive properties for its digital use.

### BACKGROUND OF THE INVENTION

At present there are different products relating to the homage and memory of living beings, whether people or pets, especially when they have died. Said products are traditionally created by hair, teeth and other parts or tissues, belonging to the loved one to be remembered.

However, so far no composition has been developed based on the "essence" of the loved one in various forms or presentations.

### DESCRIPTION OF THE INVENTION

The present invention provides a composition characterized in that it comprises resin, metal selected from among Au, Ag, Pt and Rh, graphene and a biological sample of a subject, whether alive or dead, where said biological sample is dry. Said composition has the purpose of the manufacturing of an emotional object or locket.

The advantage obtained by the use of the emotional composition of the present invention compared with the others described in the state of the art is the capacity for personalization and the homogeneous appearance both of the composition and the object formed with the composition. Another advantage consists of the preservation of biological material of the subject, alive or dead, which forms part of the emotional link between said subject and the person who wears or possesses said lyophilized emotional composition. Furthermore, said composition has conductivity which allows its use as a digital element.

Therefore, a first aspect of the invention relates to a lyophilized emotional composition characterized in that it comprises:
(a) metal powder, where said metal is selected from among Au, Ag, Pt and Rh;
(b) graphene;
(c) silica;
(d) resin; and
(e) a biological sample of a subject, where said biological sample is dry.

In a preferred embodiment, the subject is a live subject.

In a preferred embodiment, the subject is a dead subject.

In a preferred embodiment, the lyophilized emotional composition is characterized in that the resin has a particle size less than 0.002 millimetres and where the biological sample has a particle size less than 0.02 mm, this preferred embodiment is suitable for obtaining a suitable composition with optimum particle sizes for its use as 3D printer ink, since with this particle size of the lyophilized emotional composition it is thus optimized for its extrusion at pressures of 4 bars from the pressurized chamber of a 3D printer.

In another preferred embodiment, the lyophilized emotional composition is characterized in that the resin is selected from the list comprising: epoxy resins, photosensitive resins and any combination of the above.

In a more preferred embodiment, the lyophilized emotional composition is characterized in that the resin is epoxy resin.

In another preferred embodiment, the lyophilized emotional composition is characterized in that the biological sample is selected from the list comprising: micronized hair, micronized tissue, micronized cornea, tissue from micronized cornea, micronized nails, micronized teeth, DNA lyophilized with silica, non-lyophilized DNA and any combination of the above.

In a more preferred embodiment, the lyophilized emotional composition is characterized in that the biological sample is micronized hair.

In another more preferred embodiment, the lyophilized emotional composition is characterized in that the biological sample is DNA lyophilized with silica.

In another more preferred embodiment, the lyophilized emotional composition is characterized in that the biological sample is non-lyophilized DNA.

In another preferred embodiment, the lyophilized emotional composition is characterized in that the sum of the volume of the resin, the silicon, and the biological sample is a fraction of 1/3 of the volume with respect to the volume of the final composition.

In another preferred embodiment, the lyophilized emotional composition is characterized in that the metal powder is a fraction of 1/3 of the volume with respect to the volume of the final composition.

In another preferred embodiment, the lyophilized emotional composition is characterized in that the metal powder is of Au and more preferably the volume of the Au powder is a fraction of 1/3 in the composition with respect to the total volume of the final composition.

In another preferred embodiment, the lyophilized emotional composition is characterized in that the volume of the graphene is a fraction of 1/3 with respect to the total volume of the final composition.

In another preferred embodiment, the lyophilized emotional composition is characterized in that the subject is human or animal.

In another more preferred embodiment, the emotional composition is in solid form and also comprises an outer layer (c) of enamel, where said outer layer of enamel is suitable for the case that the composition is used as a ceramic figure or element or three-dimensional figure or element obtained from 3D printing. This enamel is added so that the pores generated during said firing process are occluded with said enamel giving uniformity and rigidity to the final ceramic element or figure or three-dimensional figure or element obtained from 3D printing. This enamel is added after firing in a conventional furnace of the emotional composition at a temperature of between 800 ºC and 900 ºC and prior to a second firing in a conventional furnace at a temperature of between 1000 ºC and 1060 ºC.

An even more preferred embodiment of the composition, where the layer of enamel is a resin selected from the following list: epoxy resin, photosensitive resin or any combination of the above.

Another aspect of the present invention relates to an element or figure comprising a composition as described in the present invention.

Another aspect of the present invention relates to an element or figure comprising a composition comprising an outer layer of enamel, which is preferably a resin selected from the following list: epoxy resin, photosensitive resin or any combination of the above, and which is mounted on a support.

A preferred embodiment of the present invention is the element or figure where said element or figure is three-dimensional.

Another aspect of the present invention relates to the use of the composition described above as ink of a 3D printer to obtain a three-dimensional element or figure. Preferably, the three-dimensional element or figure is a locket.

Another aspect of the present invention relates to the use of the aforementioned composition for the manufacturing of a locket.

A more preferred embodiment is the use of the three-dimensional element or figure, or ceramic figure, already enamelled, mounted on the corresponding supports.

In the present invention "lyophilized emotional composition" is understood as all compositions comprising a biological sample isolated from a live or dead (deceased) subject, and which is preferably lyophilized at a temperature of between -90 ºC and -40 ºC.

In the present invention "biological sample" is understood as all those samples of wet and dry biological material obtained from members or part of members, tissues, hair, nails, teeth, DNA or lyophilized DNA, from any human or animal, or material from a plant, which can be human, animal or plant, whether alive or dead. This biological sample is dry and ground until a micrometric size. This micronizing can be performed by a cryogenic grinder, this process is advantageous since given the organic characteristics of the sample, it is an affordable and profitable way to achieve the base of the composition of the invention and of the final object with the suitable calibre and appearance characteristics.

In the present invention "DNA lyophilized with silica" is understood as all biological samples where the DNA is extracted by techniques known by a person skilled in the art and which later undergo a lyophilization process in silica. The lyophilization process consists of subjecting a sample to a quick cooling process and to a vacuum achieving that the water directly evaporates and dehydrates the sample. With this process we manage to maintain the sample with its organoleptic conditions for long periods of time at the same time as we generate the bonding of the different particles that form the substance; this bond is electrostatic, the so-called Van Der Waals forces and which include attractions between atoms, molecules and surfaces. They differ from the covalent bond and the ionic bond in that they are caused by correlations in the fluctuating polarizations of nearby particles (due to quantum dynamics).

The final composition is frozen at a temperature between -90°C and -70 ºC, and it is introduced in the "Cryodos" apparatus (a laboratory lyophilizer) where it is subjected to a vacuum process at temperatures less than -90 ºC to -40 °C during a period of 5-6 hours. With this process, we perform an electrostatic bond of all the particles that form the composition.

Throughout the description and the claims, the word "comprises" and its variants are not intended to exclude other technical characteristics, additives, components or steps. For persons skilled in the art, other objects, advantages and characteristics of the invention will be inferred in part from the description and in part from the practice of the invention. The following examples are provided by way of illustration and it is not intended that they are limitative of the present invention.

### PREFERRED EMBODIMENT OF THE INVENTION

A detailed explanation is provided below of an example of preferred embodiment of the object of the present invention.

### Preparation of a figure with a base of resin, silica, gold powder, graphene, and a biological sample

The biological sample is DNA lyophilized in silica, which will follow a previous process described with the object of obtaining a material from which functional DNA can be recovered if necessary, without prejudice of using non-functional DNA substances for purposes other than healthcare or the conservation of genetic material through the lyophilized emotional composition object of the present utility model.

For the variant of genetic material, what we want to achieve is a DNA conservation and display material; since it is not possible to see the DNA with the naked eye, it is necessary to subject it to a series of molecular processes to manage to detect it. What we do is treat the DNA in two stages with microparticles of silica, graphene and gold powder with an amino acid buffer, such as arginine or glycine, among others, during hours and with stirring in the first of the stages. These amino acids help in bonding the silica to the DNA. We remove the excess silica particles or centrifuged DNA and wash with the same amino acid buffer. The resulting product is a liquid sample of the DNA bound to the silica, to the naked eye we see a whitish liquid. In this point the second stage starts, consisting of mixing the liquid with graphene and gold microparticles, generating a greyish substance with gold hues; the graphene and the gold have the function of giving structural consistency and conductivity to the definitive material.

With the bonding process of the DNA to the silica, what we want to achieve is a DNA conservation and display material; since it is not possible to see the DNA with the naked eye, it is necessary to subject it to a series of molecular processes to manage to detect it.

The first thing to do is treat at least 100 ng of DNA with 2 mg of silica microparticles in 150µl of amino acid buffer, such as arginine or glycine. This amino acid buffer must be at a concentration of 100mM in a Tris-EDTA solution and adjusted to pH 5. We stir the mixture at 10 Hz during 2 hours at ambient temperature in the "Thermo-shaker TS-100". In this process, the buffer's amino acids help the bonding of the silica to the DNA. After 2 hours we centrifuge the DNA samples with silica immersed in the amino acid buffer, leaving the DNA bound to the silica particles.

The graphene is added to said liquid in a proportion of 1/3 of the final volume, the gold powder in a proportion of 1/3 of the final volume and the resin until completing 1/3 of final volume together with the DNA and the silicon, and it is mixed by stirring; the end substance is the liquid we subject to the lyophilization process.

The DNA deposited in silica is previously frozen at around -80°C and we introduce it in the "Cryodos" apparatus (a laboratory lyophilizer, where we subject it to a vacuum process at a temperature of -80°C during a period of 5.5 hours. With this process we perform an electrostatic bond of all the particles that form the base material generating the lyophilized emotional composition.

The final piece is mounted on the corresponding supports.

## Claims

1. Lyophilized composition, **characterized in that** it comprises:
(a) metal powder, where said metal is selected from among Au, Ag, Pt and Rh;
(b) graphene;
(c) silica;
(d) resin; and
(e) a biological sample of a subject, where said biological sample is dry.

2. Composition according to claim 1, where the subject is a live subject.

3. Composition according to claim 1, where the subject is a dead subject.

4. Composition according to any of claims 1 to 3, where the resin is selected from the list comprising: epoxy resins, photosensitive resins and any combination of the above.

5. Composition according to any of claims 1 to 4, where the metal powder is Au.

6. Composition according to any of claims 1 to 5, where the resin is epoxy resin.

7. Composition according to any of claims 1 to 6, where the biological sample is selected from the list comprising: micronized hair, micronized tissue, micronized cornea, tissue from micronized cornea, micronized nails, micronized teeth, DNA lyophilized with silica, non-lyophilized DNA, plant tissue and combinations thereof.

8. Composition according to any of claims 1 to 7, where the biological sample is micronized hair.

9. Composition according to any of claims 1 to 7, where the biological sample is DNA lyophilized with silica.

10. Composition according to any of claims 1 to 7, where the biological sample is non-lyophilized DNA.

11. Composition according to any of claims 1 to 10, where the sum of the volume of the resin, the silicon, and the biological sample is a fraction of 1/3 of the volume with respect to the volume of the final composition.

12. Composition according to any of claims 1 to 11, where the metal powder is a fraction of 1/3 of the volume with respect to the volume of the final composition.

13. Composition according to any of claims 1 to 12, where the graphene is a fraction of 1/3 of the volume with respect to the volume of the final composition.

14. Composition according to any of claims 1, 3 to 13, where the dead subject is human or animal.

15. Composition according to any of claims 1 to 14, where said composition comprises an outer layer of enamel.

16. Composition according to claim 15, where the layer of enamel is a resin selected from the following list: epoxy resin, photosensitive resin or any combination of the above.

17. Element or figure comprising a composition according to any of claims 1 to 16.

18. Element or figure comprising a composition according to any of claims 15 or 16 and which is mounted on a support.

19. Element or figure according to any of claims 17 or 18, where the element or figure is three-dimensional.

20. Use of the composition described according to any of claims 1 to 16, for the manufacturing of a locket.

21. Use of the composition described according to any of claims 1 to 14, as ink of a 3D printer to obtain a three-dimensional element or figure.

22. Use according to claim 21, where the three-dimensional element or figure is a locket.

23. Use according to any of claims 19 or 21, where the three-dimensional element or figure or ceramic figure, already enamelled, is mounted on the corresponding supports.
